# EUROPÄISCHE PATENTSCHRIFT

(11) **EP 3 151 903 B1**
(45) Veröffentlichungstag und Bekanntmachung des Hinweises auf die Patenterteilung: **25.05.2022**
(21) Anmeldenummer: 15725078.8
(22) Anmeldetag: 03.06.2015
(51) Int. Cl.: A61M 25/10, A61F 2/95

(54) **BALLONKATHETER MIT EINER EINFÜHRHILFE FÜR EINEN FÜHRUNGSDRAHT**
BALLOON CATHETER WITH AN INSERTION AID FOR A GUIDE WIRE
CATHÉTER À BALLONNET DOTÉ D'UNE AIDE D'INTRODUCTION POUR UN FIL DE GUIDAGE

(30) Priorität: 06.06.2014 EP 14171435
(43) Veröffentlichungstag der Anmeldung: 12.04.2017
(73) Patentinhaber: B. Braun Melsungen AG, 34212 Melsungen (DE)
(72) Erfinder: SCHARSCHMIDT, Karl-Heinz, 12157 Berlin (DE); CALISSE, Jorge, 12203 Berlin (DE); WÖBKEN, Henning, 15837 Mahlow (DE); GÜNTHER, Peter, 12557 Berlin (DE)
(74) Vertreter: Patentanwälte Ruff, Wilhelm, Beier, Dauster & Partner mbB
(86) Internationale Anmeldenummer: PCT/EP2015/062415
(87) Internationale Veröffentlichungsnummer: WO 2015/185648

(56) Entgegenhaltungen:
- EP-A1- 2 361 651
- CH-B1- 700 826
- US-A- 5 893 868
- US-A- 6 110 146
- US-A1- 2011 213 451

## Beschreibung

Die Erfindung betrifft in erster Linie einen Ballonkatheter, insbesondere einen Ballonkatheter mit Stent, wobei der Katheter eine Schutzhülle aufweist, die den Ballon und gegebenenfalls auch den Stent mindestens teilweise umschließt. Weiter betrifft die Erfindung auch eine Schutzhülle für einen Ballonkatheter sowie ein Verfahren zum Einführen eines Führungsdrahts in das Innere eines Ballonkatheters.

Katheter werden in der Medizin für verschiedene diagnostische oder therapeutische Zwecke verwendet. Sie werden in der Regel in Hohlorgane, wie Magen, Darm, aber auch über die Blutbahn in Blutgefäße oder in das Herz eingeführt, um auf diese Weise vor Ort zu diagnostizieren oder zu therapieren.

Sogenannte Ballonkatheter finden insbesondere bei der Perkutanen Transluminalen Angioplastie (PTA) und bei der Perkutanen Transluminalen Koronaren Angioplastie (PTCA) Verwendung. Unter diesen Begriffen sind dabei Techniken zur Erweiterung eines verengten oder zur Wiedereröffnung eines verschlossenen (in der Regel peripheren) Blutgefäßes bzw. eines Herzkranzgefäßes zusammengefasst.

Die entsprechenden Techniken sind im Folgenden nicht einschränkend, sondern nur beispielhaft am Fall der PTCA näher beschrieben.

Üblicherweise wird bei einer PTCA ein sogenannter Führungskatheter, in der Regel ausgehend über einen Zugang zur Leistenarterie, entgegen dem Blutstrom zum Herzen geführt und dort in die Öffnung der Herzkranzarterie eingelegt. Durch das Innere des Führungskatheters wird dann der Ballonkatheter vorgeschoben. Am distalen Ende des Ballonkatheters befindet sich der Ballon, mit dessen Hilfe die Engstelle des betreffenden Herzkranzgefäßes aufgedehnt wird. Häufig wird im Anschluss an die Aufdehnung mit dem Ballon eine Gefäßstütze eingesetzt, die das Risiko eines erneuten Gefäßverschlusses herabsetzen soll. Bei diesen Gefäßstützen handelt es sich um dünne Drahtgeflechte (sogenannte Stents), die auf einen Ballonkatheter in zusammengefaltetem Zustand aufgesetzt werden. Durch die Aufdehnung des Ballons wird der Stent an die Gefäßwand gepresst und dieses Gefäß dadurch offen gehalten.

Um die PTCA ausführen zu können, muss die Gefäßverengung (unter Röntgenkontrolle) mit Hilfe eines dünnen, biegsamen Drahts, des sogenannten Führungsdrahts, passiert werden.

Dieser Führungsdraht dient als eine Art Führungsschiene, entlang derer der Ballonkatheter später in die Verengung hineinbewegt wird.

Solche Führungsdrähte sind sehr dünn und besitzen in der Regel Durchmesser von weit unterhalb von 1 mm.

Um wie zuletzt geschildert vorzugehen, muss vor Einführen des Ballonkatheters in den Körper der entsprechende Führungsdraht (distal) in den Ballonkatheter selbst eingeführt werden. Nur dann kann der Führungsdraht ja seine Funktion als Führungsschiene für den Ballonkatheter erfüllen.

Dieses Einführen des Führungsdrahts in den Ballonkatheter gestaltet sich schwierig, da der Führungsdraht mit den genannten geringen Durchmessern in das Katheterinnere mit einem nur geringfügig größeren Durchmesser eingeführt werden muss. Beispielsweise ist es in solchen Fällen in der Kardiologie erforderlich, einen Führungsdraht mit einem Durchmesser von 0,36 mm in einen Schlauch/Katheter mit ca. 0,4 mm Innendurchmesser einzuführen (einzufädeln). Dies ist insbesondere bei schlechten Lichtverhältnissen kompliziert und setzt eine gute Sehschärfe sowie eine ruhige Hand voraus.

Als weiteres Problem kommt hinzu, dass Ballonkatheter zwar in der Regel eine Schutzhülle aufweisen, die den Ballon selbst und einen gegebenenfalls vorhandenen Stent vor Beschädigung schützt. Übliche Schutzhüllen müssen jedoch vor dem Einführen des Führungsdrahtes zwangsläufig entfernt werden, so dass nun durch die notwendige Handhabung der Ballon und gegebenenfalls der Stent beim Einführen des Führungsdrahtes entweder durch diesen selbst oder anderweitig beschädigt werden können. Dies ist insbesondere bei Ballons und Stents von Bedeutung, die zusätzlich beschichtet sind. Hier kann es sich beispielsweise um sogenannte funktionale Beschichtungen handeln, die die beschichteten Oberflächen für spezielle Anwendungen geeignet machen, wie eine hydrophile Beschichtung, oder um Beschichtungen aus oder mit Wirkstoffen, insbesondere Medikamenten. In solchen Fällen kann die Beschichtung zumindest teilweise beschädigt oder abgelöst werden.

In diesem Zusammenhang soll kurz erläutert werden, wie der Anwender eines Ballonkatheters üblicherweise vorgeht, um einen Führungsdraht in den entsprechenden Katheter einzuführen.

Ist keine Einführhilfe vorhanden, so nimmt der Anwender den Ballonkatheter in der Weise in eine Hand, dass das Führungsende, d.h. das vordere Ende des Katheters bzw. die Spitze des Katheters auf der Innenseite der Kuppe eines Fingers, insbesondere des Zeigefingers aufliegt.

Dann wird mit der anderen Hand das (hintere) Ende des Führungsdrahtes ebenfalls auf die entsprechende Innenseite der Fingerkuppe aufgelegt, möglichst genau in axialer Verlängerung des Katheters.

Dann schiebt der Anwender das genannte Ende des Führungsdrahtes auf den Ballonkatheter zu und versucht, den Führungsdraht in die Öffnung des Ballonkatheters einzuschieben/einzufädeln.

Wie bereits erläutert, hat diese Vorgehensweise beispielsweise den Nachteil, dass das Auflegen eines ggf. beschichteten Ballonkatheters, insbesondere mit Medikamenten beschichteten Ballonkatheters auf die Fingerkuppe unweigerlich zu einem Teilabrieb dieser Beschichtung im Bereich des Fingers führt. Damit wird die Medikamentendosis auf dem Ballonkatheter in unerwünschter und nicht vorhersagbarer Weise verringert.

Außerdem benötigt das geschilderte Einschieben/Einfädeln eine ruhige Hand und ein gutes Auge, insbesondere da die Öffnung, die beim Einschieben getroffen werden muss, vergleichsweise klein ist.

Aus dem Stand der Technik sind sogenannte Einführhilfen für Führungsdrähte von Kathetern bekannt. Es handelt sich hierbei um separate Vorrichtungen, die distal auf den Katheter aufgesetzt werden müssen und durch ihre Konstruktion das Einführen des Führungsdrahtes in den Katheter erleichtert sollen. Als Beispiele wird hier auf die folgenden Patentveröffentlichungen verwiesen: US-A-5,978,699, WO-A2-2008/036842 und WO-A1-2012/115753.

Allerdings befinden sich die beschriebenen Einführhilfen nach ihrer Verwendung selbst ebenfalls auf dem Führungsdraht zwischen dem Katheter und dem Patienten, so dass man die Einführhilfen zwangsläufig wieder entfernen können muss, ohne den Führungsdraht aus dem Katheter (oder aus dem Patienten) ziehen zu müssen.

Außerdem lösen solche separaten Einführhilfen auch nicht das Problem einer möglichen Beschädigung von Ballon und gegebenenfalls Stent. Auch für die Verwendung dieser Einführhilfen muss nämlich eine gegebenenfalls vorhandene Schutzhülle vor der Einführung des Führungsdrahts entfernt werden. Das Risiko einer Beschädigung von Ballon oder Stent, insbesondere das Risiko einer Beschädigung von Beschichtungen auf Ballon oder Stent, ist somit auch hier vorhanden.

Aus der CH 700 826 B1 ist ein Katheter mit angeformter Katheterummantelung bekannt, wobei die Katheterummantelung in einem Bereich vor der Katheterspitze einen trichterförmigen Einführkanal als Einführhilfe für einen Führungsdraht aufweist.

Die Erfindung stellt sich dementsprechend die Aufgabe, das Risiko einer Beschädigung von Ballon oder Stent bei einem Ballonkatheter gegenüber bisher bekannten Ausführungen zu minimieren. Gleichzeitig soll das Einführen des Führungsdrahts in den Katheter selbst möglichst einfach sein. Unnötige oder umständliche Handgriffe für den Arzt sollen, insbesondere bei der Durchführung einer PTA oder einer PTCA, entfallen.

Diese Aufgabe wird gelöst durch die Schutzhülle mit den Merkmalen des Anspruchs 1 und den Ballonkatheter mit den Merkmalen des Anspruchs 12. Die Erfindung umfasst auch das in Anspruch 13 beschriebene Verfahren zum Einführen eines Führungsdrahts in einen Ballonkatheter. Bevorzugte Ausführungen dieser Schutzhülle sind in den abhängigen Produktansprüchen definiert.

Unter "Ballonkatheter" wird erfindungsgemäß ein meist aus Kunststoff gefertigter Katheter verstanden, der einen entfaltbaren Ballon aufweist, welcher sich in der Regel an der Spitze des Katheters befindet. Dabei kann der Ballon gegebenenfalls auch (funktional) beschichtet sein, vorzugsweise mit mindestens einem Medikament oder mindestens einem anderen Wirkstoff. Ein Ballonkatheter weist häufig mindestens zwei oder auch mehrere Hohlräume (Lumen) auf, die in Längsrichtung des Ballonkatheters verlaufen. Ein inneres Lumen nimmt dabei den Führungsdraht auf, ein weiter außen angeordnetes Lumen weist das Inflationsmedium für den Ballon auf. Ballonkatheter finden Verwendung vor allem in der Angioplastie, d. h. bei der Erweiterung verengter Gefäße.

Der Begriff "Stent" bezeichnet (auch im Sinne des deutschen Begriffs "Gefäßstütze") ein medizinisches Implantat, das in Hohlorgane eingebracht wird, um diese offen zu halten. Solche Stents sind meist gitterförmig als Röhrchen aufgebaut, die aus Metall und/oder Kunststoff bestehen können. Auch solche Stents können funktional und insbesondere mit Medikamenten oder anderen Wirkstoffen beschichtet sein.

Unter dem Begriff der "Schutzhülle" sollen nach der Erfindung alle Gegenstände nach Art einer Hülle oder Umhüllung zusammengefasst werden, die den Ballonkatheter (unmittelbar) mindestens teilweise umschließen und somit die entsprechenden Teile des Ballonkatheters gegen äußere Einflüsse abschirmen oder schützen. Vorzugsweise ist der Ballonkatheter, und gegebenenfalls auch ein zusätzlich vorgesehener Stent von der Schutzhülle im Wesentlichen vollständig, insbesondere vollständig, d.h. zur Gänze, umschlossen.

Als "Führungsdraht" wird gemäß der Erfindung ein Draht (oder gegebenenfalls auch ein Gegenstand mit gleicher Funktion wie bspw. ein Federrohr oder dgl.) bezeichnet, der nach Art einer Führungsschiene für die Bewegung eines Ballonkatheters innerhalb eines Hohlorgans, insbesondere innerhalb eines Gefäßes, dienen kann.

Die in dieser Anmeldung verwendeten Begriffe "distal" und "proximal" sollen im Sinne einer Lage- und Richtungsbezeichnung, die auf den Anwender des Katheters, d.h. beispielsweise den Arzt, bezogen ist, verstanden werden. Dabei bedeutet "distal" - entfernt - und "proximal" bedeutet - nahe liegend -. Wenn dementsprechend von einem distalen Ende der Schutzhülle oder des Katheters die Rede ist, so bedeutet dies, dass - bezogen auf den Funktionszustand des entsprechenden Gegenstands - das fragliche Ende das vordere Ende der Schutzhülle oder des Katheters bildet, das "vom Anwender (Arzt) entfernt" ist. Der Anwender (Arzt) fasst den Katheter dann beispielsweise am proximalen (hinteren) Ende der Schutzhülle bzw. des Katheters an.

Bei einem Ballonkatheter der vorliegenden Erfindung, der vorzugsweise zusätzlich einen Stent aufweist, ist eine Schutzhülle vorgesehen, die den Ballon und gegebenenfalls auch den Stent mindestens teilweise, vorzugsweise im Wesentlichen vollständig umschließt. Erfindungsgemäß ist dabei eine Einführhilfe, d. h. eine Einrichtung, die das Einführen eines Führungsdrahts in den Ballonkatheter, also in das entsprechende Lumen des Ballonkatheters, erleichtert, in das distale Ende der Schutzhülle integriert.

Im Ergebnis bedeutet dies, dass im Unterschied zum Stand der Technik keine separate Einführhilfe benötigt wird, sondern dass eine solche Einführhilfe bereits am distalen Ende der Schutzhülle vorhanden ist. Man kann dementsprechend auch von erfindungsgemäßen Ausführungen sprechen, bei denen die Einführhilfe an der Schutzhülle bereits vormontiert ist oder einstückig mit dieser Schutzhülle ausgebildet ist.

Um den Führungsdraht dann tatsächlich mit Hilfe der Einführhilfe in das Innere des Katheters einführen zu können, muss an der in die Schutzhülle integrierten Einführhilfe entweder eine entsprechende Öffnung geschaffen werden oder diese Öffnung kann vorzugsweise an der Einführhilfe bereits vorgesehen sein. Im letzteren Fall sind dann keine weiteren, gegebenenfalls aufwendigen Maßnahmen erforderlich, um eine solche Öffnung an der Einführhilfe erst zu schaffen. In diesem Zusammenhang ist es auch möglich, dass an der Einführhilfe Mittel vorgesehen sind, die eine Ausbildung der Öffnung auf einfache Weise ermöglichen. So können beispielsweise Sollbruchstellen oder Perforationen oder Einschnitte in der Schutzhülle vorhanden sein, mit deren Hilfe ein Führungsdraht die Schutzhülle an der entsprechenden Stelle einfach durchstoßen und damit die für das Einführen nötige Öffnung selbständig schaffen kann.

Im Zusammenhang mit den zuletzt gemachten Ausführungen kann an der Schutzhülle gegebenenfalls auch ein Gegenstand oder ein Werkzeug vorgesehen sein, das entweder eine entsprechende Öffnung an der Einführhilfe offen hält oder das Erzeugen einer solchen Öffnung erleichtert. Ein solches Mittel kann auch (gegebenenfalls zusätzlich zu der bereits beschriebenen Funktion im Zusammenhang mit der Öffnung an der Einführhilfe) dazu dienen, ein unabsichtliches Entfernen der Schutzhülle vom Katheter zu verhindern.

Bei dem genannten Mittel kann es sich beispielsweise um ein Werkzeug nach Art eines Stiletts handeln, das zum einen die Öffnung an der Schutzhülle für den Führungsdraht offen halten oder öffnen kann, und zum anderen ein unabsichtliches Entfernen der Schutzhülle vom Katheter, beispielsweise durch ein Abrutschen, verhindert.

In Weiterbildung ist die in die Schutzhülle integrierte Einführhilfe vorzugsweise kanalartig oder rohrartig ausgebildet. Dadurch kann der langgestreckte Führungsdraht in einfacher Weise innerhalb der Einführhilfe in Richtung auf das Innere des Katheters geführt werden. Dabei ist es weiter bevorzugt, wenn sich die Einführhilfe in Richtung der distal vorgesehenen Öffnung trichterartig erweitert, d. h. in Richtung dieser Öffnung eines größere Querschnittsfläche besitzt als in Richtung des Inneren des Katheters. Dadurch kann der Führungsdraht leichter in den kanalartigen oder rohrartigen Teil der Einführhilfe eingeführt (eingefädelt) werden.

Die Form der Querschnittsfläche einer kanalartigen oder rohrartigen Einführhilfe (einschließlich der trichterartigen Erweiterungen) ist erfindungsgemäß nicht kritisch, so dass hier beispielsweise dreieckförmige, rechteckförmige, quadratische, ovale oder beliebig anders geformte Querschnittsflächen realisiert sein können. Vorzugsweise sind die entsprechenden Querschnittsflächen kreisförmig, so dass sie der in der Regel kreisförmigen Querschnittsfläche von Führungsdrähten entsprechen.

Gemäß der Erfindung ist am distalen Ende der Einführhilfe eine Vertiefung nach Art einer Wanne ausgebildet. Ggf. kann diese Vertiefung auch an diesem Ende der Einführhilfe angeformt sein.

Diese wannenartige Vertiefung dient dazu, die Einführung des Führungsdrahtes, d.h. das beschriebene Einschieben oder Einfädeln in die entsprechende Öffnung, hier in die Öffnung der Einführhilfe (weiter) zu erleichtern. Die einseitig offene Vertiefung, d.h. die im Gebrauchszustand nach oben und auch an einer Seite offene Vertiefung, hat nämlich den Vorteil, dass der Führungsdraht in diese Vertiefung seitlich und/oder von oben her eingelegt werden kann. Dadurch wird der Führungsdraht gegenüber der Öffnung, in die er eingeschoben/eingefädelt werden soll, quasi automatisch richtig positioniert. Ein axiales Heranführen des Führungsdrahts an die entsprechende Öffnung mit einem genauen Treffen der entsprechenden Öffnung wird auf diese Weise vermieden.

Bei den beschriebenen bevorzugten Ausführungsformen kann die beschriebene Vertiefung grundsätzlich eine beliebige Querschnittsfläche besitzen. Insbesondere ist dabei eine kreissegmentförmige, vorzugsweise eine halbkreisförmige Querschnittsfläche, oder eine V-förmige Querschnittsfläche bevorzugt. Bei solchen Querschnittsflächen wird sich der Führungsdraht nach dem Einbringen/Einlegen in die Vertiefung im unteren Bereich der Vertiefung anordnen, und ist dementsprechend mit der dadurch erreichten Führung in Richtung auf die Öffnung bewegbar.

Mit einer solchen Vertiefung lässt sich die "Einfangfläche" für den Führungsdraht, insbesondere bei den bevorzugt genannten Querschnittsflächen für die Vertiefung, auch gegenüber konusförmigen Trichtern bei gleicher Materialmenge erheblich vergrößeren, da eine solche Vertiefung insgesamt flacher ausgeformt sein kann. Zudem könnte auch ein geometrischer Versatz der Vertiefung in axialer Richtung (gegenüber der übrigen Geometrie der Einführhilfe) akzeptiert werden, da ein solcher Versatz den Einschiebvorgang/Einfädelvorgang nicht negativ beeinflusst.

Weiter ist es nach der Erfindung bei den genannten bevorzugten Ausführungsformen von Vorteil, wenn sich die Querschnittsfläche der Vertiefung zum distalen Ende der Einführhilfe hin vergrößert, insbesondere stetig (kontinuierlich) vergrößert. Insbesondere bei einem seitlichen Einlegen des Führungsdrahts in die Vertiefung wird dadurch das Eindringen des Führungsdrahts in die Einführhilfe noch weiter erleichtert.

Wie bereits beschrieben, erleichtert die gemäß der Erfindung vorgesehene Vertiefung das Einschieben/Einfädeln des Führungsdrahts in die Öffnung der Einführhilfe. Dementsprechend ist es weiter bevorzugt, wenn diese Öffnung mit dem tiefsten Punkt der Querschnittsfläche der Vertiefung fluchtet, so dass der in die Vertiefung eingebrachte Führungsdraht zwangsläufig in diese Öffnung hineingleitet, was durch eine entsprechende Ausgestaltung des entsprechenden Endes der Vertiefung hin zur Öffnung unterstützt werden kann. Dementsprechend liegt dann auch die Öffnung des Ballonkatheters, in die der Führungsdraht dann aus der Einführhilfe heraus hineingeführt wird, vorzugsweise auf Höhe dieses tiefsten Punkts der Querschnittsfläche der Vertiefung.

Bei den bevorzugten Ausführungsformen des erfindungsgemäßen Ballonkatheters mit Vertiefung am distalen Ende der Einführhilfe ist es weiter bevorzugt, wenn diese Vertiefung gekennzeichnet, insbesondere zur Verbesserung ihrer optischen Erkennbarkeit durch den Anwender gekennzeichnet ist. Insbesondere handelt es sich bei einer solchen Kennzeichnung um eine farbliche Kennzeichnung. Diese (farbliche) Kennzeichnung erleichtert es dem Anwender die Vertiefung, in die er den Führungsdraht einlegen oder einführen soll, zu identifizieren.

Vorzugsweise ist diese Kennzeichnung, insbesondere farbliche Kennzeichnung an der Innenseite der Vertiefung vorgesehen, d.h. an der Seite der Vertiefung, mit der der Führungsdraht in Berührung kommt.

In Weiterbildung ist es bei dem erfindungsgemäßen Ballonkatheter bevorzugt, wenn die Schutzhülle proximal benachbart zur Einführhilfe mindestens einen Bereich aufweist, der gegenüber der übrigen Form der Schutzhülle abgeflacht ist. Dabei kann sich dieser abgeflachte Bereich im Gebrauchszustand der Einführhilfe mindestens an der nach unten ausgerichteten Seite der Schutzhülle befinden, d.h. auf der Seite, die beispielsweise in der beschriebenen Art mit dem Finger des Anwenders in Berührung kommt. Vorzugsweise kann sich die entsprechende Abflachung nicht nur an dieser unteren Seite der Einführhilfe, sondern auch an deren Oberseite befinden, insbesondere aufgrund der einfacheren Herstellbarkeit einer solchen abgeflachten Form.

Durch die genannte Abflachung lässt sich der entsprechende Bereich der Einführhilfe/Schutzhülle leicht auf der Fingerkuppe eines Anwenders plazieren. Der Ballonkatheter rutscht auf diese Weise auch nicht leicht von der Fingerkuppe ab. Die Handhabbarkeit des erfindungsgemäßen Ballonkatheters wird somit insgesamt verbessert.

Auch proximal, d.h. in Richtung ihres proximalen Endes, kann die Einführhilfe vorzugsweise trichterartig erweitert sein. Dadurch wird auch die Aufnahme und Festlegung des Katheters, die ja gegebenenfalls an diesem proximalen Ende der Einführhilfe erfolgt, erleichtert. Auch hier ist vorzugsweise die Form einer Querschnittsfläche der trichterartigen Erweiterung auf die die Form Querschnittsfläche des Katheters abgestimmt, wobei es sich auch hier insbesondere um kreisförmige Querschnittsflächen handelt.

Bei den erfindungsgemäß beanspruchten Ballonkathetern ist es zweckmäßig, wenn das Entfernen der Schutzhülle (erst) nach Einführen des Führungsdrahts in das Innere des Katheters erfolgt. Dementsprechend ist es erfindungsgemäß von Vorteil, wenn bei bevorzugten Ausführungsformen des erfindungsgemäßen Ballonkatheters an der Schutzhülle Mittel zum Entfernen dieser Schutzhülle vom Katheter vorgesehen sind. Derartige Mittel sind vorzugsweise in Längsrichtung der Schutzhülle (bezogen auf die Längsrichtung des Katheters und des darin eingeführten Führungsdrahtes) vorgesehen. Solche in Längsrichtung angeordneten Mittel erleichtern das Entfernen der Schutzhülle, da auf diese Weise die Schutzhülle vom Führungsdraht und/oder vom Katheter in einfacher Weise abgezogen oder abgeschält werden kann. Dies wird im Zusammenhang mit der Zeichnung noch verdeutlicht.

Vorzugsweise handelt es sich bei den Mitteln zum Entfernen der Schutzhülle vom Katheter (und/oder vom gegebenenfalls bereits eingeführten Führungsdraht) um mindestens eine Sollbruchstelle in der Schutzhülle, oder um mindestens eine Perforation in der Schutzhülle, oder um mindestens einen Einschnitt in der Schutzhülle. Mit Vorteil können auch mehrere der genannten Mittel gleichzeitig an der Schutzhülle oder in der Schutzhülle vorgesehen sein.

Allen genannten Mitteln ist gemeinsam, dass durch sie auf einfache Weise die Schutzhülle um den Katheter und gegebenenfalls um den Stent und/oder um den Führungsdraht herum geöffnet und auf einfache Weise von diesen abgenommen werden kann. Dadurch kann die Schutzhülle aus einem stabilen, mechanisch festen Material ausgebildet werden, welches den Ballon und dessen Beschichtung zuverlässig gegen eine Beschädigung schützen kann. Durch die Verwendung eines solchen festen (starren) Materials kann auch ein grundsätzlich nicht erwünschter Kontakt zwischen Schutzhülle und Ballonaußenfläche vermieden oder zumindest reduziert werden. Ein (nachträgliches) Öffnen, Aufschneiden oder dergleichen der Schutzhülle, für die Werkzeuge wie Messer, Scheren und dergleichen benötigt werden, ist nicht erforderlich.

In Weiterbildung ist bei den Ausführungsformen des erfindungsgemäßen Ballonkatheters, bei denen Mittel zum Entfernen der Schutzhülle vorgesehen sind, zusätzlich eine Art Handhabe für diese Mittel vorgesehen. Mit Hilfe dieser Handhabe, die vorzugsweise nach Art einer Lasche oder einer Kerbe ausgebildet sein kann, können die Mittel zum Entfernen der Schutzhülle auf einfachere Weise betätigt werden. Zum Beispiel kann an der entsprechenden Lasche oder Kerbe durch einen Anwender des Ballonkatheters angegriffen und auf diese Weise dann die Schutzhülle, beispielsweise anhand dort vorhandener Perforationen einfach aufgetrennt bzw. aufgerissen werden.

Bei allen Ausführungsformen der Erfindung kann die genannte Schutzhülle aus unterschiedlichen Materialien bestehen, wobei vorzugsweise Kunststoffmaterialien zum Einsatz kommen. Lediglich beispielhaft sollen hier Materialien wie Polytretrafluorethylen, Polyethylen, Polypropylen oder Polyether-Block-Amid genannt werden.

Um zu verhindern, dass der Ballonkatheter vom Anwender in den Körper des Patienten eingeführt wird, ohne die Schutzhülle vom Katheter abzunehmen, kann die Schutzhülle optisch gekennzeichnet oder eingefärbt sein, beispielsweise mit auffälligen (Signal-)Farben und/oder Mustern. Auch ein absichtlich vergrößerter Durchmesser der Schutzhülle über zumindest einen Teil ihrer Länge kann diese (Sicherungs-)Funktion erfüllen.

Schließlich kann sich der erfindungsgemäße Ballonkatheter vorzugsweise innerhalb einer sterilen Umverpackung befinden. Dementsprechend ist auch eine solche sterile Umverpackung, in der ein erfindungsgemäßer Ballonkatheter enthalten ist, Gegenstand der Erfindung.

Die Erfindung umfasst die Schutzhülle mit integrierter Einführhilfe, die bereits im Zusammenhang mit dem erfindungsgemäßen Ballonkatheter beschrieben wurde. Eine solche Schutzhülle ist für einen Ballonkatheter, insbesondere für einen Ballonkatheter mit Stent vorgesehen und sie umschließt den Ballon und gegebenenfalls auch den Stent mindestens teilweise, vorzugsweise im Wesentlichen vollständig. Erfindungsgemäß ist dabei in die Schutzhülle, insbesondere in das distale Ende der Schutzhülle eine Einführhilfe für einen Führungsdraht integriert.

In Weiterbildung kann die Schutzhülle mit Vorteil so ausgebildet sein, wie dies bereits im Zusammenhang mit den bevorzugten Ausführungsformen des erfindungsgemäßen Ballonkatheters beschrieben wurde. Dies bezieht sich somit auf die Merkmale der Öffnung in der Einführhilfe zum Einführen des Führungsdrahts, die kanalartige oder rohrartige Ausbildung der Einführungshilfe, gegebenenfalls mit trichterartiger Erweiterung distal und/oder proximal. In diesem Zusammenhang wird insbesondere auch auf alle Ausführungsformen der Einführhilfe mit der erfindungsgemäßen wannen- oder der beispielhaften, nicht zum Gegenstand der vorliegenden Erfindung gehörigen kanalartigen Vertiefung Bezug genommen. Auch die beschriebenen Mittel zum Entfernen der Schutzhülle vom Katheter und die in diesem Zusammenhang offenbarte Handhabe sollen hiermit durch Bezugnahme auch Teil der Beschreibung im Zusammenhang mit den Merkmalen der Schutzhülle werden.

Schließlich umfasst die Erfindung auch ein Verfahren zum Einführen eines Führungsdrahts in das Innere eines Ballonkatheters, insbesondere eines Ballonkatheters mit Stent. Dabei wird ein Ballonkatheter bzw. ein Ballonkatheter mit Stent bereitgestellt, wobei dieser Ballonkatheter eine Schutzhülle aufweist, die den Ballon und gegebenenfalls auch den Stent mindestens teilweise, im Wesentlichen vollständig umschließt. In das distale Ende der Schutzhülle ist zusätzlich die erfindungsgemäße Einführhilfe integriert. Dann wird verfahrensgemäß der Führungsdraht distal über die Einführhilfe in die Schutzhülle eingeführt.

Die beschriebene Erfindung ist mit einer ganzen Reihe von Vorteilen verbunden.

So kann eine erfindungsgemäße Schutzhülle mit integrierter Einführhilfe für ganz unterschiedliche Ballonkatheter eingesetzt werden. Es kann sich dabei um Ballonkatheter mit oder ohne Stent handeln. Besonders bevorzugt ist die Verwendung der erfindungsgemäßen Schutzhülle im Zusammenhang mit beschichteten, insbesondere medikamentenbeschichteten Ballonkathetern, wobei entweder der Ballon oder (alternativ oder zusätzlich) ein gegebenenfalls vorhandener Stent beschichtet sein kann.

Ein entscheidender Vorteil der Erfindung liegt darin, dass gegenüber separaten Einführhilfen, wie sie aus dem Stand der Technik bekannt sind, im Falle der Erfindung der Ballon des Ballonkatheters oder gegebenenfalls auch ein vorhandener Stent während der Einführung des Führungsdrahtes nicht berührt/angefasst werden müssen. Bei korrekter Handhabung des erfindungsgemäßen Ballonkatheters bzw. der erfindungsgemäßen Schutzhülle ist es sogar nicht möglich, den Ballon bzw. den Stent während des Einführens/Einfädelns des Führungsdrahtes zu berühren. Die Schutzhülle mit integrierter Einführhilfe deckt, insbesondere bei bevorzugten Ausführungsformen, alle übrigen Bauteile des erfindungsgemäßen Ballonkatheters vollständig ab. Der genannte Vorteil kommt insbesondere bei beschichteten/medikamentenbeschichteten Ballonkathetern (mit oder ohne Stent) zum Tragen. Hier sollte eine Berührung während der Handhabung, d. h. im vorliegenden Fall insbesondere bei der Einführung des Führungsdrahtes, unbedingt vermieden werden. Ansonsten würden die aufgebrachten Schichten, insbesondere die aufgebrachten Medikamentenschichten beschädigt und damit die Funktion der Beschichtung zumindest beeinträchtigt, und eventuell sogar verhindert.

Als weiterer Vorteil ist zu erwähnen, dass für den Anwender der erfindungsgemäßen Ballonkatheter die Handhabung beim Einführen des Führungsdrahts deutlich vereinfacht wird. Es muss ja keine separate Einführhilfe zunächst aufgesetzt werden, um das Einführen überhaupt zu ermöglichen. Die integrierte Einführhilfe ermöglicht es, sofort mit der Einführung des Führungsdrahtes zu beginnen.

Im zuletzt genannten Zusammenhang ist auch hervorzuheben, dass die Integration der Einführhilfe in die Schutzhülle in der Regel bei der Herstellung der Schutzhülle einfach zu realisieren ist. Dementsprechend ist gegenüber der Verwendung einer separaten Einführhilfe in der Regel eine Kosteneinsparung für den Anwender verbunden.

Die genannten Vorteile und weitere Vorteile ergeben sich aus der folgenden Beschreibung im Zusammenhang mit den Zeichnungen und dem Beispiel. Dabei können die dort beschriebenen Merkmale bei der Erfindung einzeln oder in Kombination miteinander verwirklicht sein.

In der Zeichnung zeigt
- Figur 1: die schematische Darstellung einer nicht erfindungsgemäßen Schutzhülle mit integrierter Einführhilfe in Draufsicht,
- Figur 2: die schematische Darstellung einer weiteren nicht erfindungsgemäßen Schutzhülle mit integrierter Einführhilfe in Schnittansicht,
- Figur 3: die schematische Darstellung einer erfindungsgemäßen Schutzhülle, bei der die integrierte Einführhilfe eine wannenartige Vertiefung besitzt (Seitenansicht), und
- Figur 4: die schematische Darstellung der erfindungsgemäßen Schutzhülle gemäß Figur 3 (Draufsicht).

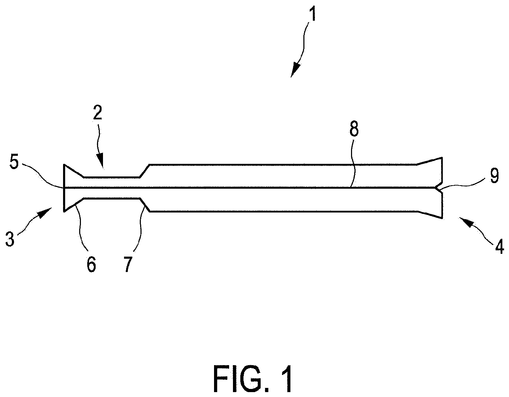

Die nicht in allen Details dargestellte Schutzhülle 1 gemäß Figur 1 weist eine integrierte Einführhilfe 2 auf. Dabei ist in Figur 1 das Bezugszeichen 3 links dem distalen Ende der Schutzhülle 1 und das Bezugszeichen 4 rechts dem proximalen Ende der Schutzhülle 1 zugeordnet.

In Übereinstimmung mit den Ausführungen in der Beschreibung wird dementsprechend ein in Figur 1 nicht dargestellter Führungsdraht von der linken Seite der Figur (distal) in das Innere der Schutzhülle 1 eingeführt. Im Inneren der Schutzhülle 1 befindet sich der eigentliche Ballonkatheter (mit oder ohne Stent), der in Figur 1 aus Gründen der Übersichtlichkeit nicht dargestellt ist. Ein derartiger Ballonkatheter ist über das proximale Ende 4 der Schutzhülle 1 in das Innere der Schutzhülle 1 eingebracht. Dieses Einbringen wird durch die am proximalen Ende 4 vorgesehene Erweiterung der Querschnittsfläche, die in Figur 1 nicht näher bezeichnet ist, erleichtert.

Die in die Schutzhülle integrierte Einführhilfe 2 besteht im Wesentlichen aus einem rohrförmigen Abschnitt, der sich in Richtung einer am distalen Ende 3 vorgesehenen Öffnung 5 nach Art eines Trichters 6 erweitert. Der rohrförmige Abschnitt mit trichterförmiger Erweiterung besitzt im Falle der Darstellung der Figur 1 eine kreisförmige Querschnittsfläche. Über die Öffnung 5 kann ein in Figur 1 nicht dargestellter Führungsdraht in das Innere der Schutzhülle 1 eingebracht werden. Die trichterförmige Erweiterung 6 gewährleistet dabei ein erleichtertes Einbringen/Einfädeln des Führungsdrahts in das Innere der rohrförmigen Einführhilfe 2.

In Richtung auf das proximale Ende 4 der Schutzhülle 1 ist die Einführhilfe 2 nach dem engeren rohrförmigen Abschnitt ebenfalls nach Art eines Trichters 7 erweitert. In der Regel ist der in Figur 1 nicht dargestellte Ballonkatheter (mit oder ohne Stent) vom proximalen Ende 4 der Schutzhülle 1 her bis an diese trichterförmige Erweiterung 7 herangeführt und dort festgelegt. Dies bedeutet, dass ein Führungsdraht nach Passieren der trichterförmigen Erweiterung 6, des (engeren) rohrförmigen Abschnitts der Einführhilfe 2, und nach Passieren der trichterförmigen Erweiterung 7 in das Innere (das inneren Lumen) des Ballonkatheters gleitet und in diesem Inneren des Ballonkatheters weiter hindurchgeführt wird. Damit ist die vorteilhafte Funktion der Einführhilfe 2, die in die Schutzhülle 1 integriert ist, durch Figur 1 deutlich dargestellt.

Weiter ist in Längsrichtung der Schutzhülle 1 ein Einschnitt 8 vorgesehen, der das Material der Schutzhülle 1 (mit integrierter Einführhilfe 2) längs durchschneidet. Dabei kann sich dieser Einschnitt über die gesamte Dicke des Schutzhüllenmaterials erstrecken oder doch zumindest über einen wesentlichen Teil dieser Materialdicke. Im ersteren Fall ist das Material der Schutzhülle also vollständig durchschnitten, und wird beispielsweise durch die Materialspannung des entsprechenden Kunststoffmaterials (noch) mechanisch zusammengehalten. Im letzteren Fall bedarf es dann aufgrund der fast vollständigen Durchtrennung des Materials nur noch einer geringen Kraft, um ein vollständiges Öffnen der Schutzhülle entlang des Einschnitts zu bewirken. Eine vergleichbare Lösung kann auch durch Perforationen in Längsrichtung anstelle des nicht vollständigen Einschnitts realisiert sein.

Um das Material der Schutzhülle gegebenenfalls leichter entlang des Einschnitts 8 öffnen zu können, ist am proximalen Ende 4 der Schutzhülle 1 eine Kerbe 9 vorgesehen. Diese ermöglicht es dem Benutzer des erfindungsgemäßen Ballonkatheters in einfacher Weise an der Schutzhülle anzugreifen und auf diese Weise die entlang des Einschnitts geöffnete oder noch zu öffnende Schutzhülle vom Katheter und/oder Führungsdraht abzunehmen.

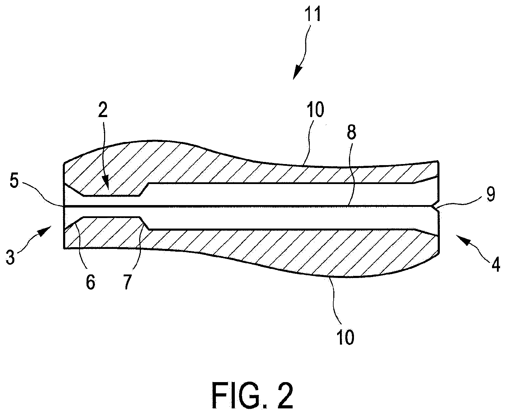

In Figur 2 ist eine weitere Ausführung einer nicht erfindungsgemäßen Schutzhülle mit integrierter Einführhilfe in einer schematischen Schnittansicht dargestellt.

Aus Gründen der Übersichtlichkeit und der Vergleichbarkeit mit Figur 1 sind die wesentlichen Komponenten der Schutzhülle 11 gemäß Figur 2 mit den gleichen Bezugszeichen wie in Figur 1 bezeichnet, beispielsweise die integrierte Einführhilfe 2 mit ihren trichterförmigen Erweiterungen 6 und 7. Auch distales Ende 3 mit der dort vorgesehenen Öffnung 5 sowie das proximale Ende 4 sind mit den gleichen Bezugszeichen versehen. Dies gilt auch für den Einschnitt 8 und die Kerbe 9, mit deren Hilfe die Schutzhülle 11 in der bereits beschriebenen Weise einfach geöffnet werden kann.

Bei der in Figur 2 dargestellten Schutzhülle 11 ist, im Vergleich zur Schutzhülle 1 von Figur 1, lediglich gezeigt, dass für die eigentliche vorteilhafte Funktion der Schutzhülle mit integrierter Einführhilfe die innere Form bzw. die Innenkontur der Schutzhülle 1 bzw. 11 sowie der integrierten Einführhilfe 2 entscheidend ist. Auf die Darstellung dieser Innenkontur ist bei der Darstellung gemäß Figur 1 in besonderer Weise abgestellt. Die Außenkontur der Schutzhülle 1 ist nicht im Detail dargestellt und kann daher bei der Ausführung gemäß Figur 1 im Wesentlichen der Form der Innenkontur entsprechen.

Bei der Ausführung gemäß Fig. 2 ist hingegen verdeutlicht, dass die äußere Form bzw. die Außenkontur der Schutzhülle 11 für deren Funktion nicht von Bedeutung ist Die in Figur 2 dargestellte Außenkontur 10 der Schutzhülle 11 kann von der dort dargestellten Innenkontur, welche der Darstellung von Figur 1 entspricht, in beliebiger Weise abweichen. Entscheidend bei der Erfindung ist, dass die Innenkontur der Schutzhülle, zusammen mit der Innenkontur der in die Schutzhülle integrierten Einführhilfe so ausgebildet ist, dass ein nicht dargestellter Führungsdraht vom distalen Ende 3 her über die Einführhilfe in das Innere der Schutzhülle eingebracht und dann weiter in den in der Schutzhülle festgelegten Ballonkatheter eingeführt werden kann.

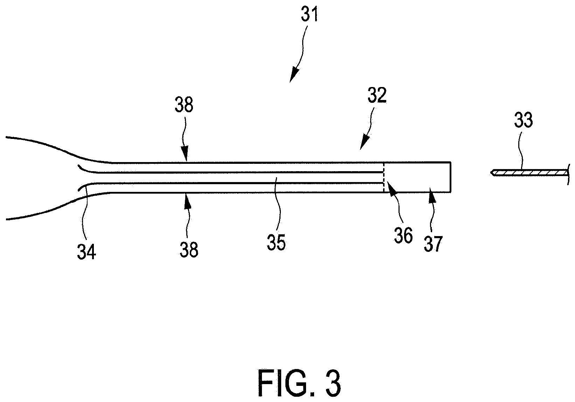

Von der erfindungsgemäßen Schutzhülle gemäß Figur 3 ist im Wesentlichen nur der Teil mit der integrierten Einführhilfe 32 dargestellt, wobei der schematisch gezeichnete Führungsdraht 33 noch nicht in die Einführhilfe 32 eingeführt ist.

Die Einführhilfe 32 der Schutzhülle 31 besitzt gemäß Figur 3 an ihrem (links dargestellten) proximalen Ende eine trichterförmige Erweiterung 34, die zur Aufnahme des Katheters, der in Figur 3 nicht dargestellt ist, vorgesehen ist. An die Öffnung 34 schließt sich ein rohrartiger Kanal 35 an, der in der distal vorgesehenen Öffnung 36 der Einführhilfe 32 endet.

Am (äußersten) distalen Ende der Einführhilfe 32 befindet sich eine wannenartige Vertiefung 37, die sowohl nach oben als auch distal (d.h. in der Figur 3 nach rechts) offen ist. In die so ausgebildete Vertiefung 37 kann der Führungsdraht entweder von oben oder (wie in Figur 3 dargestellt) von der (hier rechten) Seite eingeführt werden.

Die Querschnittsfläche der Vertiefung 37 fluchtet mit der Öffnung 36, so dass der Führungsdraht 33 nach dem Einbringen in die Vertiefung 37 in einfacher Weise in die Öffnung 36 hinein- und durch den Kanal 35 in Richtung auf die Öffnung 34 hindurch gleiten kann.

Darüber hinaus zeigt Figur 3 wie die Einführhilfe 32 der Schutzhülle 31 proximal benachbart zur Vertiefung 37, sowohl an der Oberseite als auch an der Unterseite abgeflachte Bereiche (Abflachungen) 38 aufweist, was das Einbringen des Führungsdrahts 33 in die Einführhilfe 32 für den Anwender in der bereits beschriebenen Weise erleichtert.

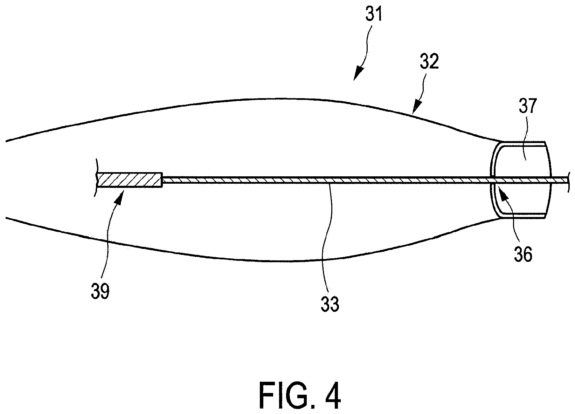

Figur 4 zeigt die erfindungsgemäße Schutzhülle 31 mit Einführhilfe 32 in Draufsicht, und zwar in dem Zustand, in welchem der Führungsdraht 33 bereits mit Hilfe der Einführhilfe 32 im Katheter plaziert ist. Gemäß Figur 4 ist der Führungsdraht 33 nach Einlegen (von der rechten Seite her; distal) durch die Öffnung 36 und den in Figur 4 nicht näher bezeichneten Kanal 35 in das distale Ende 39 des Katheters eingeführt und somit im Katheter plaziert.

Die Merkmale und Vorteile der Erfindung werden im Folgenden nochmals im Zusammenhang mit dem Beispiel beschrieben.

### Beispiel

Im Folgenden soll die Funktionsweise eines erfindungsgemäßen Ballonkatheters mit erfindungsgemäßer Schutzhülle (mit integrierter Einführhilfe) nochmals im Zusammenhang, unter Einbeziehung der Figuren 1, 3 und 4 erläutert werden.

Ausgangspunkt ist ein Ballonkatheter (mit oder ohne Stent), der sich im Inneren einer nicht erfindungsgemäßen Schutzhülle 1 (mit integrierter Einführhilfe 2) befindet. Gemäß Figur 1 ist der Ballonkatheter dabei mit seiner Spitze an der trichterförmigen Erweiterung 7 der Einführhilfe 2 angelegt. Mit Vorteil umschließt die Schutzhülle 1 den in Figur 1 nicht dargestellten Ballonkatheter vollständig.

Zur Vorbereitung der Verwendung des Ballonkatheters, beispielsweise in einer PTA oder PTCA, wird ein Führungsdraht über die am distalen Ende 3 der Einführhilfe 2 vorgesehene Öffnung 5 in das Innere der Einführhilfe 2 eingeführt. Dabei kann die Öffnung 5 an der Einführhilfe 2 entweder bereits vorhanden sein oder mit Hilfe dort vorgesehener Mittel und/oder mit Hilfe eines Werkzeugs (z. B. Stilett) eröffnet werden. Die Vertiefung 37 der Einführhilfe 32 erleichtert das Einführen des Führungsdrahts in das Innere der Einführhilfe. Nachdem der Führungsdraht die Einführhilfe passiert hat, wird er (automatisch) in das Innere (das innere Lumen) des Ballonkatheters, der sich innerhalb der Schutzhülle 1 befindet, eingeführt/eingefädelt und im Inneren des Ballonkatheters vorangeführt. Das gesamte Einführen des Führungsdrahts erfolgt in einem Zustand, in dem die Schutzhülle 1 den Ballonkatheter entweder an den empfindlichen Bauteilen oder vorzugsweise vollständig abdeckt. Eine Berührung des Ballonkatheters, einschließlich der auf dem Ballon und/oder dem Stent vorgesehener Beschichtungen, ist nicht erforderlich, oder sogar gar nicht möglich.

Nach dem Einführen des Führungsdrahts in das Innere des Ballonkatheters kann die Schutzhülle 1 vom Katheter abgenommen werden. Vorzugsweise erfolgt dies in einem Zustand, in dem der Ballonkatheter selbst über das proximale Ende 4 der Schutzhülle 1 aus dem Inneren der Schutzhülle (in proximaler Richtung) herausgezogen ist. In diesem Zustand befindet sich dann die Schutzhülle 1 nur noch über dem Führungsdraht. Dies hat zum einen den Vorteil, dass das Entfernen der Schutzhülle nicht über möglicherweise empfindlichen Teilen des Ballonkatheters (z. B. über Beschichtungen) erfolgt. Zum anderen besitzt der Führungsdraht selbst ja einen geringeren Durchmesser als der Ballonkatheter, so dass ein Abnehmen der Schutzhülle vom Führungsdraht aufgrund seiner Abmessungen einfacher ist.

Zum eigentlichen Entfernen der Schutzhülle 1 wird dann die Schutzhülle entlang des Einschnitts 8 geöffnet und zumindest teilweise aufgeklappt/aufgefächert. Dies wird durch die an der Schutzhülle 1 vorgesehene Kerbe 9 erleichtert. Dann kann die Schutzhülle 1 über den Führungsdraht abgenommen werden, ohne dass der Ballonkatheter selbst von diesem Abnehmen betroffen ist.

## Patentansprüche

1. Schutzhülle (1; 11; 31) für einen Ballonkatheter, insbesondere für einen Ballonkatheter mit Stent, die den Ballon und gegebenenfalls vorzugsweise auch den Stent mindestens teilweise umschließt, wobei in das distale Ende (3) der Schutzhülle (1; 11; 31) eine Einführhilfe (2; 32) für einen Führungsdraht (33) integriert ist, **dadurch gekennzeichnet, dass** am distalen Ende der Einführhilfe (32) eine wannenartige Vertiefung (37) ausgebildet oder dort angeformt ist, die sowohl nach oben als auch distal offen ist.

2. Schutzhülle nach Anspruch 1, **dadurch gekennzeichnet, dass** distal an der Einführhilfe (2) eine Öffnung (5) zum Einführen des Führungsdrahts in das Innere des Katheters vorgesehen ist.

3. Schutzhülle nach Anspruch 1 oder Anspruch 2, **dadurch gekennzeichnet, dass** die Einführhilfe (2; 32) kanalartig oder rohrartig ausgebildet ist, wobei sich die Einführhilfe (2) vorzugsweise in Richtung der distal vorgesehenen Öffnung (5) trichterartig (6) erweitert.

4. Schutzhülle nach einem der vorhergehenden Ansprüche, **dadurch gekennzeichnet, dass** die Vertiefung (37) eine kreissegmentförmige, insbesondere eine halbkreisförmige Querschnittsfläche, oder eine V-förmige Querschnittsfläche besitzt.

5. Schutzhülle nach einem der vorhergehenden Ansprüche, **dadurch gekennzeichnet, dass** sich die Querschnittsfläche der Vertiefung (37) zum distalen Ende der Einführhilfe (32) hin vergrößert, insbesondere stetig vergrößert.

6. Schutzhülle nach einem der vorhergehenden Ansprüche, **dadurch gekennzeichnet, dass** die Querschnittsfläche der Vertiefung (37) an ihrem proximalen Ende mit der Öffnung in der Einführhilfe (32) fluchtet.

7. Schutzhülle nach einem der vorhergehenden Ansprüche, **dadurch gekennzeichnet, dass** die Vertiefung (37) zur Verbesserung ihrer optischen Erkennbarkeit gekennzeichnet, insbesondere farblich gekennzeichnet ist, wobei vorzugsweise die Kennzeichnung, insbesondere die farbliche Kennzeichnung an der Innenseite der Vertiefung (37) vorgesehen ist.

8. Schutzhülle nach einem der vorhergehenden Ansprüche, **dadurch gekennzeichnet, dass** die Schutzhülle (1; 11; 31) proximal benachbart zur Einführhilfe (2; 32) mindestens einen gegenüber der übrigen Form der Schutzhülle abgeflachten Bereich aufweist.

9. Schutzhülle nach einem der vorhergehenden Ansprüche, **dadurch gekennzeichnet, dass** sich die Einführhilfe (2; 32) proximal (4) trichterartig (7) erweitert, zur Aufnahme und Festlegung des Katheters in der Einführhilfe.

10. Schutzhülle nach einem der vorhergehenden Ansprüche, **dadurch gekennzeichnet, dass** an der Schutzhülle (1; 11), insbesondere in deren Längsrichtung, Mittel (8) zum Entfernen der Schutzhülle vorgesehen sind, wobei vorzugsweise es sich bei den Mitteln (8) um mindestens eine Sollbruchstelle, um mindestens eine Perforation und/oder um mindestens einen Einschnitt (8) in der Schutzhülle (1; 11) handelt.

11. Schutzhülle nach Anspruch 10, **dadurch gekennzeichnet, dass** mindestens eine Handhabe (9) für die Mittel (8) zum Entfernen der Schutzhülle (1; 11) vorgesehen ist, vorzugsweise nach Art einer Lasche oder einer Kerbe (9).

12. Ballonkatheter, insbesondere Ballonkatheter mit Stent, wobei der Katheter eine Schutzhülle nach einem der vorhergehenden Ansprüche aufweist, die den Ballon und gegebenenfalls vorzugsweise auch den Stent mindestens teilweise umschließt.

13. Verfahren zum Einführen eines Führungsdrahts (33) in das Innere eines Ballonkatheters, insbesondere eines Ballonkatheters mit Stent, wobei ein Ballonkatheter, insbesondere ein Ballonkatheter mit Stent bereitgestellt wird, wobei der Ballonkatheter eine Schutzhülle (1; 11; 31) aufweist, die den Ballon und gegebenenfalls vorzugsweise auch den Stent mindestens teilweise umschließt, und wobei in das distale Ende (3) der Schutzhülle (1; 11; 31) eine Einführhilfe (2; 32) integriert ist, und dann der Führungsdraht (33) distal in die Schutzhülle (1; 11; 31) eingeführt wird, **dadurch gekennzeichnet, dass** am distalen Ende der Einführhilfe (32) eine wannenartige Vertiefung (37) ausgebildet oder dort angeformt ist, die sowohl nach oben als auch distal offen ist.

## Claims

1. Protective sleeve (1; 11; 31) for a balloon catheter, in particular for a balloon catheter with stent, which sleeve at least partially surrounds the balloon and optionally preferably also the stent, wherein an insertion aid (2; 32) for a guide wire (33) is integrated in the distal end (3) of the protective sleeve (1; 11; 31),
**characterized in that**
at the distal end of the insertion aid (32) a trough-like recess (37) is provided or molded thereon, which is open both at the top and also distally.

2. Protective sleeve according to claim 1, **characterized in that** an opening (5) for insertion of the guide wire into the interior of the catheter is provided distally on the insertion aid (2).

3. Protective sleeve according to claim 1 or claim 2, **characterized in that** the insertion aid (2; 32) has a duct-like or tubular design, wherein the insertion aid (2) preferably widens like a funnel (6) in the direction of the opening (5) provided at the distal end.

4. Protective sleeve according to any of the preceding claims, **characterized in that** the recess (37) has a cross-sectional surface area in the shape of a segment of a circle, in particular in the shape of a semicircle, or has a V-shaped cross-sectional surface area.

5. Protective sleeve according to any of the preceding claims, **characterized in that** the cross-sectional surface area of the recess (37) increases, in particular increases continuously, toward the distal end of the insertion aid (32).

6. Protective sleeve according to any of the preceding claims, **characterized in that** the cross-sectional surface area of the recess (37) is flush, at its proximal end, with the opening in the insertion aid (32).

7. Protective sleeve according to any of the preceding claims, **characterized in that** the recess (37) is coded, in particular color-coded, in order to improve its discernibility to the eye, wherein preferably the coding, in particular the color coding, is provided on the inner face of the recess (37).

8. Protective sleeve according to any of the preceding claims, **characterized in that** the protective sleeve (1; 11; 31) has, proximally adjacent to the insertion aid (2; 32), at least one area that is flattened in relation to the shape of the rest of the protective sleeve.

9. Protective sleeve according to any of the preceding claims, **characterized in that** the insertion aid (2; 32) widens proximally (4) like a funnel (7), in order to receive and secure the catheter in the insertion aid.

10. Protective sleeve according to any of the preceding claims, **characterized in that** on the protective sleeve (1; 11), in particular in the longitudinal direction thereof, means (8) for removal of the protective sleeve are provided, wherein preferably the means (8) are in the form of at least one predetermined breaking point, at least one perforation and/or at least one incision (8) in the protective sleeve (1; 11).

11. Protective sleeve according to claim 10, **characterized in that** at least one handle (9) for the means (8) for removal of the protective sleeve (1; 11) is provided, preferably in the manner of a tab or a notch (9).

12. Balloon catheter, in particular a balloon catheter with stent, wherein the catheter has a protective sleeve according to any of the preceding claims, which at least partially surrounds the balloon and optionally preferably also the stent.

13. Method for inserting a guide wire (33) into the interior of a balloon catheter, in particular of a balloon catheter with stent, wherein a balloon catheter, in particular a balloon catheter with stent, is made available, wherein the balloon catheter has a protective sleeve (1; 11; 31), which at least partially surrounds the balloon and optionally preferably also the stent, and wherein an insertion aid (2; 32) is integrated in the distal end (3) of the protective sleeve(1; 11; 31), and the guide wire (33) is then inserted distally into the protective sleeve (1; 11; 31),
**characterized in that**
at the distal end of the insertion aid (32) a trough-like recess (37) is provided or molded thereon, which is open both at the top and also distally.

## Revendications

1. Manchon de protection (1 ; 11 ; 31) destiné à un cathéter à ballonnet, en particulier un cathéter à ballonnet muni d'un stent, lequel manchon de protection enferme au moins partiellement le ballonnet et, le cas échéant, de préférence également le stent, un auxiliaire d'insertion (2 ; 32) destiné à un fil de guidage (33) étant intégré dans l'extrémité distale (3) du manchon de protection (1 ; 11 ; 31), **caractérisé en ce qu'**une dépression (37) en forme de cuvette, qui est ouverte aussi bien vers le haut que du côté distal, est ménagée ou conformée à l'extrémité distale de l'auxiliaire d'insertion (32).

2. Manchon protecteur selon la revendication 1, **caractérisé en ce qu'**une ouverture (5) destinée à insérer le fil de guidage à l'intérieur du cathéter est ménagée du côté distal sur l'auxiliaire d'insertion (2) .

3. Manchon de protection selon la revendication 1 ou la revendication 2, **caractérisé en ce que** l'auxiliaire d'insertion (2 ; 32) est conçu sous la forme d'un canal ou d'un tube, l'auxiliaire d'insertion (2) s'étendant en forme d'entonnoir (6) de préférence en direction de l'ouverture (5) prévue du côté distal.

4. Manchon de protection selon l'une des revendications précédentes, **caractérisé en ce que** l'évidement (37) a une aire en coupe transversale en forme de segment circulaire, notamment en forme de demi-cercle, ou une aire en coupe transversale en forme de V.

5. Manchon de protection selon l'une des revendications précédentes, **caractérisé en ce que** l'aire en coupe transversale de la dépression (37) augmente vers l'extrémité distale de l'auxiliaire d'insertion (32), en particulier augmente de manière continue.

6. Manchon de protection selon l'une des revendications précédentes, **caractérisé en ce que** l'aire en coupe transversale de la dépression (37) est alignée à son extrémité proximale avec l'ouverture de l'auxiliaire d'insertion (32).

7. Manchon de protection selon l'une des revendications précédentes, **caractérisé en ce que** l'évidement (37) est caractérisé de manière à améliorer sa capacité à être détecté visuellement, notamment est **caractérisé par** une couleur, la caractérisation, en particulier la caractérisation par une couleur, étant de préférence prévue du côté intérieur de la dépression (37) .

8. Manchon de protection selon l'une des revendications précédentes, **caractérisé en ce que** le manchon de protection (1 ; 11 ; 31) comporte, du côté proximal de manière adjacente à l'auxiliaire d'insertion (2 ; 32), au moins une zone aplatie par rapport à la forme restante du manchon de protection.

9. Manchon protecteur selon l'une des revendications précédentes, **caractérisé en ce que** l'auxiliaire d'insertion (2 ; 32) s'élargit du côté proximal (4) à la manière d'un entonnoir (7) pour recevoir et fixer le cathéter dans l'auxiliaire d'insertion.

10. Manchon de protection selon l'une des revendications précédentes, **caractérisé en ce que** des moyens (8) de retrait du manchon de protection sont prévus sur le manchon de protection (1 ; 11), notamment dans sa direction longitudinale, les moyens (8) étant de préférence au moins un point de rupture, au moins une perforation et/ou au moins une incision (8) dans le manchon de protection (1 ; 11).

11. Manchon de protection selon la revendication 10, **caractérisé en ce qu'**au moins une poignée (9) destinée aux moyens (8) de retrait du manchon de protection (1 ; 11) est prévue, de préférence à la manière d'une languette ou d'une encoche (9).

12. Cathéter à ballonnet, en particulier cathéter à ballonnet muni d'un stent, le cathéter comportant un manchon de protection selon l'une des revendications précédentes, qui enferme au moins partiellement le ballonnet et, le cas échéant, de préférence également le stent.

13. Procédé d'insertion d'un fil de guidage (33) à l'intérieur d'un cathéter à ballonnet, en particulier d'un cathéter à ballonnet muni d'un stent, un cathéter à ballonnet, en particulier un cathéter à ballonnet muni d'un stent, étant prévu, le cathéter à ballonnet comportant un manchon de protection (1 ; 11 ; 31) qui enferme au moins partiellement le ballonnet et, le cas échéant, de préférence également le stent, et un auxiliaire d'insertion (2 ; 32) étant intégré dans l'extrémité distale (3) du manchon de protection (1 ; 11 ; 31), et le fil de guidage (33) étant ensuite inséré du côté distal dans le manchon de protection (1 ; 11 ; 31), **caractérisé en ce qu'**une dépression (37) en forme de cuvette, qui est ouverte aussi bien vers le haut que du côté distal, est ménagée ou conformée à l'extrémité distale de l'auxiliaire d'insertion (32).
